# EUROPEAN PATENT SPECIFICATION

(11) **EP 2 087 868 B1**
(45) Date of publication and mention of the grant of the patent: **29.06.2011**
(21) Application number: 08425066.1
(22) Date of filing: 05.02.2008
(51) Int. Cl.: A61F 13/15

(54) **Method and machine for manufacturing sanitary pads for ladies, and sanitary pad obtained using said method**
Verfahren und Maschine für die Herstellung von Damenbinden und auf diese Weise hergestellte Damenbinde
Procédé et machine pour fabriquer des tampons hygiéniques pour femmes, et tampon hygiénique obtenu grâce à ce procédé

(43) Date of publication of application: 12.08.2009
(73) Proprietor: Fameccanica.Data S.p.A., 66020 Sambuceto di S. Giovanni Teatino (Chieti) (IT)
(72) Inventor: Cianci, Enio Giovanni, 66020 Sambuceto di S. Giovanni Teatino (Chieti) (IT)
(74) Representative: Marchitelli, Mauro

(56) References cited:
- EP-A- 1 078 619
- US-A1- 2003 051 802
- US-A1- 2004 040 650
- US-A1- 2004 087 425
- US-A1- 2007 213 678

## Description

### Field of the invention

The present invention relates to sanitary pads for ladies. The invention regards specifically a method and a machine for manufacturing sanitary pads having an elongated central body and two flaps that extend on opposite sides of the main body and that in use can be folded towards the rear part of the main body. The invention moreover regards a sanitary pad obtained using said method.

### Description of the prior art

The techniques of production of sanitary pads may be divided into two categories indicated in the sector as "machine-direction" and "cross-direction".

The technique of production of the machine-direction type envisages forming a moving chain of semifinished products, in which the semifinished products are oriented with their longitudinal axis aligned to the direction of movement.

According to the cross-direction technique, a moving chain of semifinished products is formed in which the semifinished products are set with their longitudinal axis transverse to the direction of movement.

The cross-direction technique is more advantageous as compared to the machine-direction technique since, given the same speed of advance of the chain of semifinished products, it enables hourly production rates to be obtained that are approximately three times higher or else, given the same hourly production rates, it enables speeds of advance approximately three times lower to be obtained. This is due to the fact that the width of the products is approximately one third of the length so that by getting the products to advance according to the cross-direction mode there is a higher productivity per hour given the same speed of advance of the chain of semifinished products.

Even though the cross-direction production technique is more advantageous, this technique is not currently used for manufacturing sanitary pads with lateral flaps. The need to fold the flaps along lines transverse to the direction of feed of the products involves in fact constructional complications which render inadvisable the use of the cross-direction technique for manufacturing these products.

The current state of the art consequently envisages the use of the machine-direction technique for manufacturing sanitary pads with transverse flaps, as disclosed for instance in EP-A-1078619, US-A-2004/0087425 and US-A-2004/0040650. US-A-2003/0051802 discloses a method for manufacturing shaped components for absorbent articles and US-A-2007/0213678 discloses an apparatus and a method for manufacturing several distinct disposable absorbent articles on a single machine.

US-A-4285343 describes a sanitary pad with an elongated central body and with transverse flaps that extend laterally from the central body. The flaps can be formed as an integral part of the central body or can be formed separately and fixed to the longitudinal edges of the central body. The lines of joining between the central body and the flaps are flexible so that the flaps can be folded on the top part of the central body for packaging purposes and can be folded towards the rear part of the central body when the absorbent pad is used.

### Object and summary of the invention

The object of the present invention is to provide a method and a machine for manufacturing sanitary pads with transverse flaps according to a cross-direction technology.

According to the present invention, said object is achieved by a method and a machine presenting the characteristics forming the subject of Claims 1 and 4.

The present invention envisages creating a moving chain of semifinished products without flaps oriented in a direction transverse to the direction of feed of the chain and producing, separately from the chain of semifinished products without flaps, a chain of elements forming the flaps that is set alongside the chain of semifinished products without flaps whilst this advances continuously in the working direction. The elements forming the flaps are then fixed to corresponding products without flaps by means of the same perimetral weld that fixes the various components of the sanitary pads to one another in a definitive way.

As will emerge clearly from the following description, the solution described herein enables all the advantages typical of the cross-direction technology to be obtained without the problems and complications that would derive from the need to fold the flaps in a direction transverse to the direction of movement of the products.

An appreciable advantage of the solution described herein is that the same machine can produce indifferently sanitary pads without flaps and sanitary pads with flaps, without requiring modifications or operations of setting-up.

The claims form an integral part of the technical teaching provided herein.

### Brief description of the annexed drawings

The present solution will now be described in detail with reference to the annexed drawings, which are provided purely by way of non-limiting example and in which:
- Figure 1 is a plan view of a sanitary pad with transverse flaps according to the solution described herein;
- Figure 2 is a plan view of the sanitary pad of Figure 1 with the flaps open;
- Figure 3 is an exploded perspective view of the sanitary pad according to the solution described herein;
- Figure 4 is a schematic exploded cross-sectional view of the sanitary pad of Figure 3;
- Figures 5 and 6 are enlarged schematic sections according to the lines V-V and VI-VI of Figures 1 and 2, respectively;
- Figure 7 is a schematic side view of a machine for manufacturing the sanitary pads illustrated in Figure 1; and
- Figures 8 and 9 are schematic plan views illustrating the method of formation of the sanitary pads according to the solution described herein.

### Detailed description of an example of embodiment

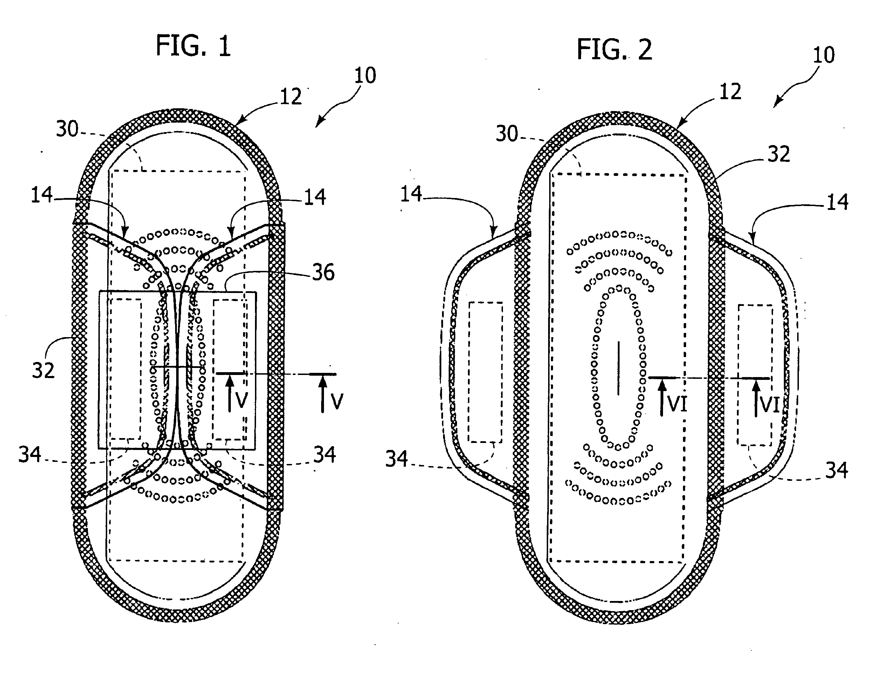

With reference to Figures 1-4, designated by 10 is a sanitary pad comprising an elongated central body 12 and two transverse flaps 14 that extend on opposite longitudinal sides of the central body 12. In a way in itself known, in the commercially available packs, the flaps 14 are folded on the top face of the central body 12 (the side that is to come into contact with the body of the user). In use, the flaps 14 can be opened as illustrated in Figure 2 and folded against the rear face of the central body 12. In use, in a known way, the central body 12 is positioned on the inside of a garment of underclothing, and the flaps 14 are then folded and made to adhere on the outside of the garment.

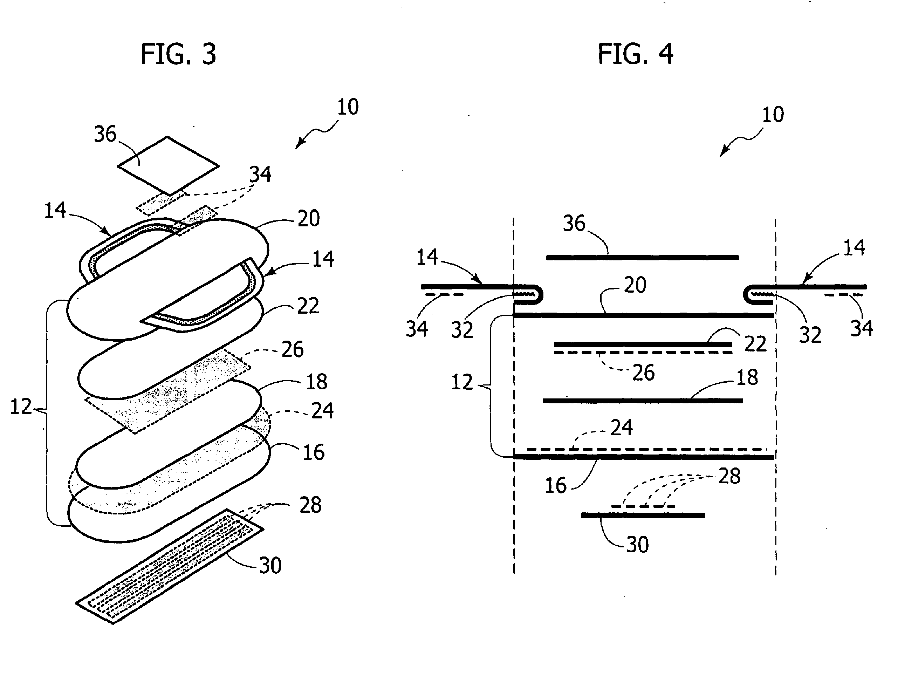

With reference in particular to Figures 3 and 4, the central body 12 has a stratified structure comprising a backsheet 16, an absorbent pad 18, a topsheet 20 and a secondary topsheet 22. The absorbent pad 18 can be fixed to the topsheet 16 by means of a layer of glue 24. The secondary topsheet 22 is set between the topsheet 20 and the absorbent pad 18 and can be fixed to the pad 18 by means of a layer of glue 26. On the rear side of the backsheet 16 a layer of pressure-sensitive glue 28 is applied, covered by a removable protective sheet 30. The structure of the central body 12 closely corresponds to the structure of a sanitary pad without flaps of a known type. The materials used for making the central body 12 are well known to a person skilled in the sector and do not call for a detailed description.

The flaps 14 are fixed on the top surface of the topsheet 20 by means of a weld designated as a whole by 32 in Figure 4. The flaps 14 are provided with respective layers of pressure-sensitive glue 34, applied on which is a removable protective strip 36, which, in addition to protecting the layers of glue 34, has also the purpose of keeping the flaps 14 in the closed position illustrated in Figure 1.

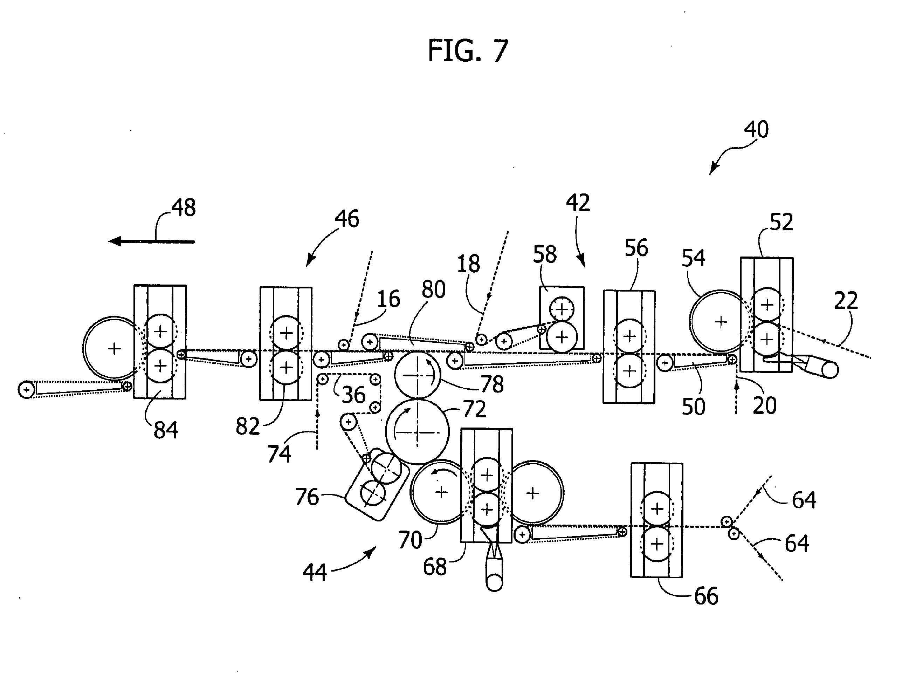

With reference to Figure 7, designated by 40 is an automatic machine for manufacturing the sanitary pads described previously. The machine 40 comprises a first section 42, in which the formation of a chain of semifinished products without flaps takes place, a second section 44 for the formation of a chain of elements forming the flaps, and a third section 46 for welding and cutting the finished products after application of the chain of elements forming the flaps to the chain of semifinished products without flaps. The chain of semifinished products moves continuously through the sections 42 and 46 in the working direction indicated by the arrow 48. In the moving chain, the semifinished products are oriented with their longitudinal axis transverse to the direction of feed 48, according to a typical cross-direction arrangement.

A continuous web of the material forming the topsheet 20 is fed at the input of the section 42 to a conveyor belt 50. A continuous web of the material constituting the secondary topsheet 22 is fed to a cutting unit 52. The web 22 is cut according to the profile of the secondary topsheet 22. After cutting, the various topsheets 22 are picked up by a transfer wheel 54 and deposited on the top surface of the topsheet 20. Next, the topsheet 20 and the secondary topsheet 22 are subjected to an operation of embossing in a unit 56.

A continuous web of the material forming the absorbent pad 18 is fed to a cutting unit 58 that cuts the continuous web according to the profile of the absorbent pad 18 and applies the absorbent pad 18 on the top surface of the secondary topsheet 22.

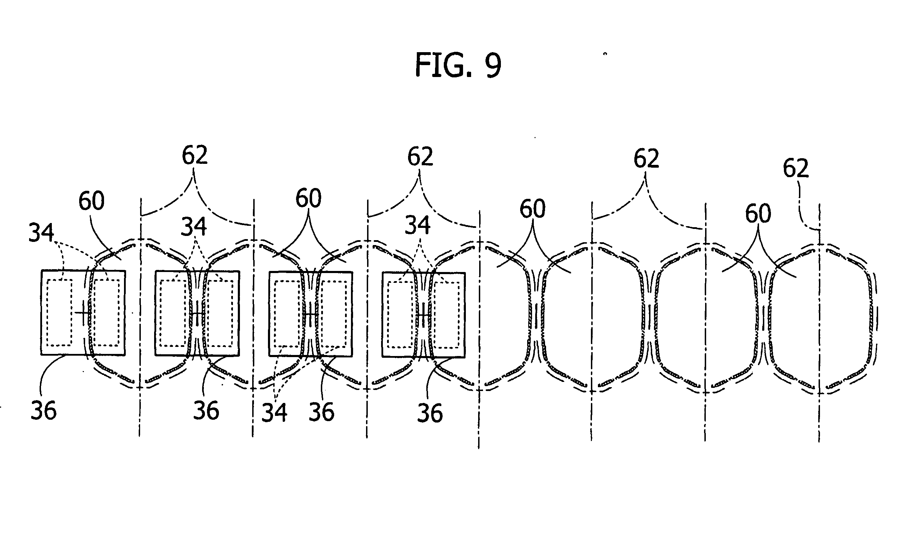

In parallel, a continuous chain of elements forming the flaps is formed in the section 44 of the machine 40. In Figure 9, the elements forming the flaps are designated by 60. Each of said elements is symmetrical with respect to a transverse axis 62 and has a shape corresponding to that of two flaps 14 set alongside one another along the respective lines of joining to the topsheet 20. Various elements forming the flaps 60 are separated from one another by means of a perimetral cutting operation. The various elements 60 are joined to one another by means of lengths of strip 36, each of which extends astride of two adjacent elements 60. The lengths of strip 36 are applied to the elements 60 by means of layers of pressure-sensitive glue 34.

With reference to Figure 7, the chain of elements forming the flaps 60 is formed starting from two continuous webs 64 that are welded to one another in a welding unit 66 and that subsequently are subjected to a perimetral cutting operation in a cutting unit 68. After cutting in the unit 68, a succession of elements 60 is obtained, detached from one another, which are picked up continuously by a first transfer wheel 70. The elements forming the flaps 60 are transferred onto a second transfer wheel 72 on which application of the lengths of strip 36 to the moving array of elements forming the flaps 60 occurs. The continuous strip 36 is fed in the point designated by 74. A unit 74 applies the pressure-sensitive glue on the strip 36 and cuts the strip 36 into a plurality of lengths separate from one another. Said lengths are then applied on the surface facing outwards of the elements 60 whilst these transit on the outer surface of the wheel 72. The chain formed by the elements 60 and by the lengths of strip 36 is then picked up by a third transfer wheel 78, which applies the chain of elements forming the flaps 60 on the side of the topsheet 20 facing downwards whilst the latter advances continuously in the working direction indicated by the arrow 48. The application of the elements forming the flaps 60 on the continuous chain of semifinished products is preferably performed in an area corresponding to a suction belt 80. At output from the belt 80, application of the backsheet 16 on the top surface of the chain of semifinished products is performed. Then, the chain of semifinished products passes through a welding unit 82 and, subsequently, through a cutting unit 84. The cutting unit 84 separates the individual finished sanitary pads from one another. Next, the layer of paper 30 with the pressure-sensitive glue is applied to the sanitary pads on the outer side of the backsheet 16, and the sanitary pads are packaged according to techniques in themselves known.

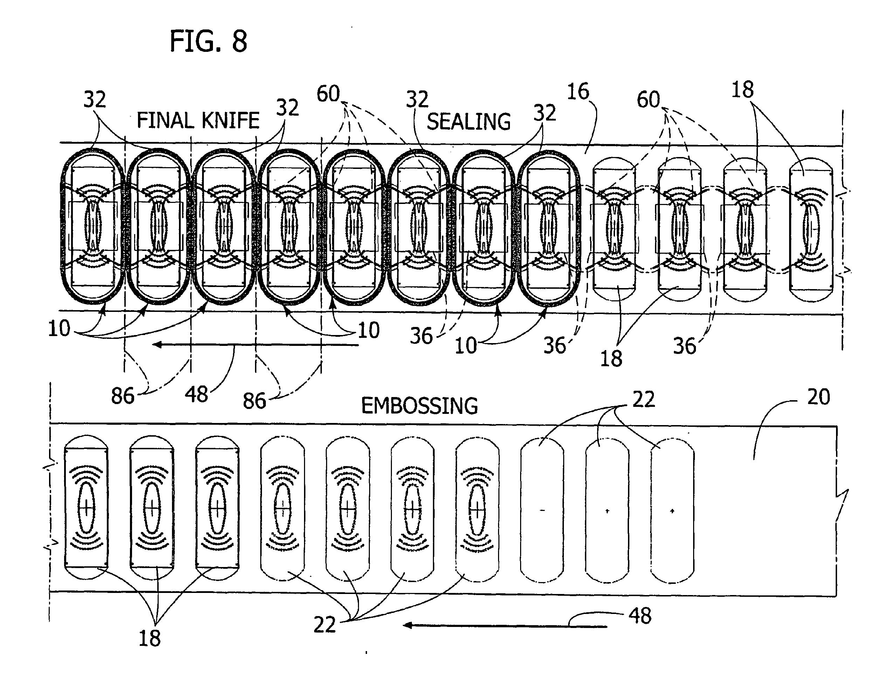

Figure 8 illustrates schematically the sequence of operations that lead to the formation of the finished sanitary pads. The topsheet 20 functions as supporting base on which various components are each time added as described previously, with the various components set transversely with respect to the direction of feed. In the welding step performed in the welding unit 82, the perimetral weld 32 is made, which fixes the topsheet 20 to the backsheet 16. With this weld, the elements forming the flaps 60 on the outer surface of the topsheet 20 are also welded. After the perimetral weld 32 is made, in the unit 84, a cut is performed along lines designated by 86. The cutting lines coincide with the axes of symmetry 62 of the elements forming the flaps 60. Following upon this cutting operation, the individual sanitary pads 10 are separated from one another, and each element forming the flaps 60 is separated into two symmetrical parts forming two flaps 14 of two adjacent sanitary pads 10.

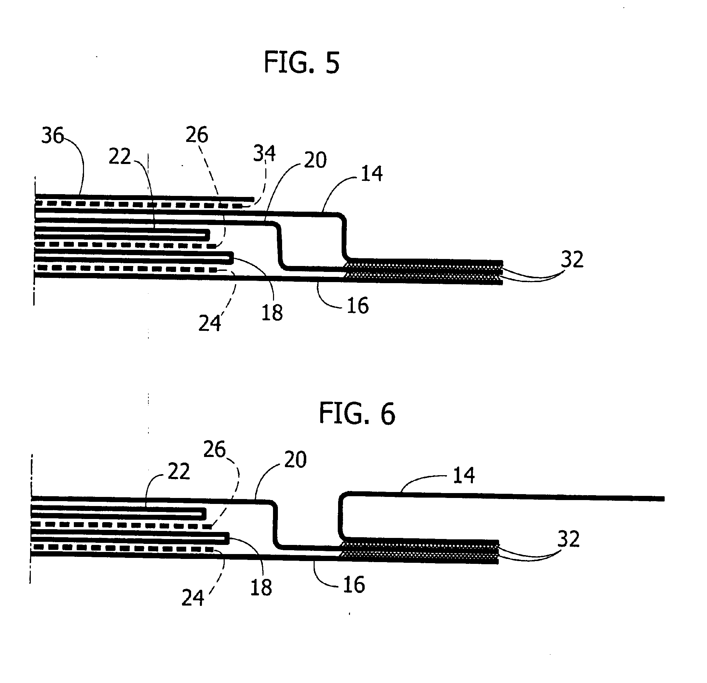

With reference to Figures 5 and 6, in each finished sanitary pad the outer perimetral edges of the topsheet 20 and of the backsheet 16 extend outwards beyond the outer edges of the absorbent pad 18 and of the secondary topsheet 22. Each flap 14 is fixed to the outer face of the topsheet 20 by means of the same perimetral weld 32 that joins the perimetral edges of the topsheet 20 and of the backsheet 16 to one another.

From the foregoing description, it is evident that with the solution described herein it is possible to produce sanitary pads with transverse flaps with the typical modality of a cross-direction process, in which the chain of semifinished products advances with the semifinished products set transversely with respect to the direction of movement. The machine described herein also enables production of sanitary pads without flaps. In fact, it is sufficient to deactivate the section 44 for producing with the same machine sanitary pads without transverse flaps.

The solution described herein enables a considerable reduction in the waste, expecially in view of the fact that the flaps are prepared separately and then applied to the chain of semifinished products without flaps. In this way, on the chain of semifinished products, the individual semifinished products can be set in positions very close to one another, reducing the free spaces between the products that would constitute waste material.

## Claims

1. A method for manufacturing sanitary pads with an elongated central body (12) and with transverse flaps (14) that extend on opposite longitudinal sides of the central body (12), said method being **characterized in that** it comprises the steps of:
- forming a moving chain of semi-finished products without flaps, with the semifinished products oriented in a direction transverse to the direction of feed (48) of the chain, the step of forming a moving chain of semi-finished products without flaps including: feeding a continuous web forming a topsheet (20), setting on said continuous web a plurality of secondary topsheets (22), setting a plurality of absorbent pads (18) on said topsheets (22),
- producing, separately from said chain of semifinished products without flaps, a chain of elements forming the flaps (60),
- applying said chain of elements forming the flaps (60) on a face of said continuous web forming the topsheet (20) opposite to the face on which said secondary topsheets (22) and said absorbent pads (18) have previously been applied,
- applying on said absorbent pads and on said topsheet (20) a continuous sheet forming a backsheet (16),
- fixing the chain of elements forming the flaps (60) to the chain of semi-finished products without flaps whilst this moves continuously in said direction of feed (48) by making a perimetral weld (32) between the topsheet (20) and the backsheet (16), whereby said elements forming the flaps (60) are fixed to an outer face of said topsheet (20), and
- cutting the continuous chain of semifinished products along transverse axes of symmetry (62) of said elements forming the flaps (60).

2. The method according to Claim 1, **characterized in that** said chain of elements forming the flaps (60) comprises a plurality of elements forming the flaps (60) detached from one another and a plurality of lengths of strip (36), each of which is fixed to two elements forming the adjacent flaps (60) by means of respective layers of pressure-sensitive glue (34).

3. The method according to Claim 1 or Claim 2, **characterized in that** each of said elements forming the flaps (60) has a shape symmetrical with respect to a transverse axis (62), each of the two symmetrical parts of each element (60) forming a flap following upon a cut made along said axis of symmetry (62).

4. A machine for manufacturing sanitary pads with an elongated central body (12) and with transverse flaps (14) that extend laterally on opposite longitudinal sides of said central body (12), said machine being **characterized in that** it comprises a section (42), which forms a continuous chain of semifinished products without flaps advancing in a working direction (48) with the semifinished products set transversely with respect to the direction of feed (48), the machine (40) moreover comprising a section (44), which forms a continuous chain of elements forming the flaps (60) and applies said chain of elements forming the flaps (60) to said chain of semifinished products without flaps whilst the latter advances in said working direction (48).

5. A sanitary pad (10) obtained by a method according to claim 1.

6. A sanitary pad (10) according to claim 5, comprising an elongated central body (12) including a backsheet (16), an absorbent pad (18) and a topsheet (20), in which the topsheet (20) and the backsheet (16) have respective perimetral edges that extend outwards beyond the absorbent pad (18) and are fixed to one another by means of a perimetral weld (32), the sanitary pad (10) comprising two flaps (14) provided as components not integral with respect to the topsheet (20) and to the backsheet (16) and fixed to opposite longitudinal edges of the central body by means of the same weld (32) that joins the backsheet (16) and the topsheet (20) to one another.

## Patentansprüche

1. Verfahren zur Herstellung von Damenbinden mit einem lang gestreckten zentralen Körper (12) und mit quer verlaufenden Flügeln (14), die sich an entgegen gesetzten Längsseiten des zentralen Körpers (12) ausdehnen, wobei das Verfahren **dadurch gekennzeichnet ist, dass** es die folgenden Schritte aufweist:
- Formen einer sich bewegenden Kette von halbfertigen Produkten ohne Flügel, wobei die halbfertigen Produkte in eine Richtung quer zu der Richtung der Zuführung (48) der Kette ausgerichtet sind, wobei der Schritt des Formens einer sich bewegenden Kette von halbfertigen Produkten ohne Flügel, umfasst:
Zuführen einer durchgängigen Bahn, welche ein Oberblatt (20) bildet, wobei auf besagte durchgängige Bahn eine Mehrzahl von sekundären Oberblättern (22) aufgelegt werden, wobei eine Mehrzahl von saugfähigen Kissen (18) auf die Oberblätter (22) aufgelegt werden,
- Produzieren einer Kette von Elementen, welche die Flügel (60) bilden, getrennt von der Kette von halbfertigen Produkten ohne Flügel,
- Aufbringen der Kette von Elementen, welche die Flügel (60) bilden, auf eine Fläche der durchgängigen Bahn, welche das Oberblatt (20) bildet, auf der anderen Fläche, auf welche die sekundären Oberblätter (22) und die saugfähigen Kissen (18) vorher aufgebracht wurden,
- Aufbringen eines durchgängigen Blatts, welches ein Rückblatt (16) bildet, auf die saugfähigen Kissen und auf das Oberblatt (20),
- Befestigen der Kette von Elementen, welche die Flügel (60) bilden, an der Kette von halbfertigen Produkten ohne Flügel durch Herstellen einer äußeren Fügenaht (32) zwischen dem Oberblatt (20) und dem Rückblatt (16), während sich diese Anordnung kontinuierlich in der Richtung der Zuführung (48) bewegt, wobei die Elemente, welche die Flügel (60) bilden, an einer äußeren Fläche des Oberblattes (20) angeheftet sind, und
- Schneiden der durchgängigen Kette von halbfertigen Produkten entlang quer verlaufender Symmetrieachsen (62) der Elemente, welche die Flügel (60) bilden.

2. Verfahren gemäß Anspruch 1, **dadurch gekennzeichnet, dass** die Kette von Elementen, welche die Flügel (60) bilden, eine Mehrzahl von Elementen aufweisen, welche die Flügel (60) bilden, welche voneinander vereinzelt sind, und eine Mehrzahl von Längen von Streifen (36), wobei jeder Streifen an zwei Elemente, welche benachbarte Flügel (60) bilden, mithilfe von zugehörigen Schichten von druckempfindlichem Klebstoff (34) angeheftet ist.

3. Verfahren gemäß Anspruch 1 oder Anspruch 2, **dadurch gekennzeichnet, dass** jedes der Elemente, welche die Flügel (60) bilden, hinsichtlich der quer verlaufenden Achse (62) eine symmetrische Form aufweist, wobei jedes der zwei symmetrischen Teile von jedem Element (60) einen Flügel bildet, nachdem ein Schnitt entlang der besagten Symmetrieachse (62) ausgeführt wurde.

4. Maschine zur Herstellung von Damenbinden mit einem lang gezogenen zentralen Körper (12) und mit quer verlaufenden Flügeln (14), die sich an entgegen gesetzten Längsseiten des zentralen Körpers (12) seitlich ausdehnen, wobei die Maschine **dadurch gekennzeichnet ist, dass** sie einen Abschnitt (42) aufweist, der eine kontinuierliche Kette von halbfertigen Produkten ohne Flügel bildet, welche sich in eine Fertigungsrichtung (48) fortbewegen, wobei die halbfertigen Produkte in Bezug auf die Richtung der Zuführung (48) quer angeordnet sing, wobei die Maschine (40) weiterhin einen Abschnitt (44) aufweist, welcher eine kontinuierliche Kette von Elementen, welche die Flügel (60) bilden, bildet und die Kette von Elementen, welche die Flügel (60) bilden, auf die Kette von halbfertigen Produkten ohne Flügel aufbringt, während sich diese in Arbeitsrichtung (48) fortbewegt.

5. Damenbinde (10) erhalten nach einem Verfahren gemäß Anspruch 1.

6. Damenbinde (10) gemäß Anspruch 5, aufweisend einen lang gestreckten zentralen Körper (12) aufweisend ein Rückblatt (16), ein saugfähiges Kissen (18) und ein Oberblatt (20), wobei das Oberblatt (20) und das Rückblatt (16) zugehörige äußere Kanten besitzen, die sich nach außen über das saugfähige Kissen (18) hinaus erstrecken und aneinander mithilfe einer äußeren Verschweißung (32) fixiert werden, wobei die Damenbinde (10) zwei Flügel (14) aufweist, welche in Bezug auf das Oberblatt (20) und das Rückblatt (16) als nicht-integrale Komponenten bereitgestellt sind und an entgegengesetzten Längskanten des zentralen Körpers mithilfe der selben Verschweißung (32) befestigt sind, welche das Rückblatt (16) und das Oberblatt (20) aneinanderfügt.

## Revendications

1. Procédé pour fabriquer des serviettes hygiéniques avec un corps central allongé (12) et avec des rabats transversaux (14) qui s'étendent sur les côtés longitudinaux opposés du corps central (12), ledit procédé étant **caractérisé en ce qu'**il comprend les étapes consistant à :
former une chaîne mobile de produits semi-finis sans rabat, avec les produits semi-finis orientés dans une direction transversale par rapport à la direction d'alimentation (48) de la chaîne, l'étape consistant à former une chaîne mobile de produits semi-finis sans rabat comprenant les étapes consistant à :
alimenter une bande continue formant une feuille supérieure (20), placer sur ladite bande continue, une pluralité de feuilles supérieures secondaires (22), placer une pluralité de serviettes absorbantes (18) sur lesdites feuilles supérieures (22),
produire, séparément de ladite chaîne de produits semi-finis sans rabat, une chaîne d'éléments formant les rabats (60),
appliquer ladite chaîne d'éléments formant les rabats (60) sur une face de ladite bande continue formant la feuille supérieure (20) opposée à la face sur laquelle lesdites feuilles supérieures secondaires (22) et lesdites serviettes absorbantes (18) ont été préalablement appliquées,
appliquer sur lesdites serviettes absorbantes et sur ladite feuille supérieure (20), une feuille continue formant une feuille inférieure (16),
fixer la chaîne d'éléments formant les rabats (60) à la chaîne de produits semi-finis sans rabat alors que celle-ci se déplace de manière continue dans ladite direction d'alimentation (48) en réalisant une soudure périmétrale (32) entre la feuille supérieure (20) et la feuille inférieure (16), moyennant quoi lesdits éléments formant les rabats (60) sont fixés sur une face externe de ladite feuille supérieure (20), et
couper la chaîne continue de produits semi-finis le long des axes transversaux de symétrie (62) desdits éléments formant les rabats (60).

2. Procédé selon la revendication 1, **caractérisé en ce que** ladite chaîne d'éléments formant les rabats (60) comprend une pluralité d'éléments formant les rabats (60) détachés les uns des autres et une pluralité de longueurs de bande (36), dont chacune est fixée à deux éléments formant les rabats (60) adjacents au moyen de couches de colle sensible à la pression (34) respectives.

3. Procédé selon la revendication 1 ou la revendication 2, **caractérisé en ce que** chacun desdits éléments formant les rabats (60) a une forme symétrique par rapport à un axe transversal (62), chacune des deux parties symétriques de chaque élément (60) formant un rabat suite à une découpe pratiquée le long dudit axe de symétrie (62).

4. Machine pour fabriquer des serviettes hygiéniques avec un corps central allongé (12) et avec des rabats transversaux (14) qui s'étendent latéralement sur les côtés longitudinaux opposés dudit corps central (12), ladite machine étant **caractérisée en ce qu'**elle comprend une section (42) qui forme une chaîne continue de produits semi-finis sans rabat avançant dans une direction de travail (48) avec les produits semi-finis placés de manière transversale par rapport à la direction d'alimentation (48), la machine (40) comprenant de plus une section (44) qui forme une chaîne continue d'éléments formant les rabats (60) et applique ladite chaîne d'éléments formant les rabats (60) sur ladite chaîne de produits semi-finis sans rabat alors que cette dernière avance dans ladite direction de travail (48).

5. Serviette hygiénique (10) obtenue par un procédé selon la revendication 1.

6. Serviette hygiénique (10) selon la revendication 5, comprenant un corps central allongé (12) comprenant une feuille inférieure (16), une serviette absorbante (18) et une feuille supérieure (20), dans laquelle la feuille supérieure (20) et la feuille inférieure (16) ont des bords périmétraux respectifs qui s'étendent vers l'extérieur au-delà de la serviette absorbante (18) et sont fixés les uns aux autres au moyen d'une soudure périmétrale (32), la serviette hygiénique (10) comprenant deux rabats (14) prévus sous la forme de composants qui ne sont pas solidaires par rapport à la feuille supérieure (20) et par rapport à la feuille inférieure (16) et fixés sur les bords longitudinaux opposés du corps central au moyen de la même soudure (32) qui assemble la feuille inférieure (16) et la feuille supérieure (20) l'une à l'autre.
